# EUROPÄISCHE PATENTSCHRIFT

(11) **EP 1 294 322 B1**
(45) Veröffentlichungstag und Bekanntmachung des Hinweises auf die Patenterteilung: **09.02.2005**
(21) Anmeldenummer: 00938436.3
(22) Anmeldetag: 30.06.2000
(51) Int. Cl.: A61F 2/46

(54) **VORRICHTUNG ZUM INJIZIEREN VON KNOCHENZEMENT**
DEVICE FOR INJECTING BONE CEMENT
DISPOSITIF POUR INJECTER DU CIMENT OSSEUX

(43) Veröffentlichungstag der Anmeldung: 26.03.2003
(73) Patentinhaber: Synthes AG Chur, 7002 Chur (CH)
(72) Erfinder: JÄGGI, Kurt, CH-3095 Spiegel bei Bern (CH); HEINI, Paul, CH-3084 Wabern (CH)
(74) Vertreter: Lusuardi, Werther Giovanni, Dr.
(86) Internationale Anmeldenummer: PCT/CH2000/000355
(87) Internationale Veröffentlichungsnummer: WO 2002/002033

(56) Entgegenhaltungen:
- WO-A-99/49819
- US-A- 4 595 006
- US-A- 4 969 888
- US-A- 5 487 392
- US-A- 5 775 333

## Beschreibung

Die Erfindung betrifft eine Vorrichtung zum Injizieren von Knochenzement, enthaltend einen Führungsdraht und eine zumindest mit dem Innendurchmesser ihrer vorderen, axialen Mündung satt auf den Führungsdraht passende Kanüle. Dokument WO-A-99/49 819 stellt den nächsten Stand der Technik dar.

Es ist bereits bekannt, osteoporotische Knochen zur Frakturprophylaxe mit injiziertem Knochenzement zu augmentieren. Mit den bisher verwendeten Injektionskanülen mit axialer Austrittsöffnung kann die Füllung in vielen Fällen nicht mit der gewünschten Genauigkeit plaziert werden.

Es ist daher eine Aufgabe der Erfindung, eine Vorrichtung zum Injizieren von Knochenzement vorzuschlagen, bei der sich die Austrittsrichtung des Knochenzements nach dem Einführen der Kanüle in einem gewissen Bereich steuern lässt.

Gemäss einer ersten Variante der Erfindung wird diese Aufgabe dadurch gelöst, dass Verschlussmittel vorhanden sind, mit denen die vordere, axiale Mündung der Kanüle nach dem Entfernen des Führungsdrahtes verschliessbar ist und dass die Kanüle nahe der vorderen, axialen Mündung eine radiale Öffnung für den Austritt des Knochenzements aufweist.

Nach einer besonderen Ausführungsart dieser Variante ist das Verschlussmittel eine an ihrem vorderen Ende geschlossene Innenkanüle, die so in die Kanüle einführbar ist, dass ihr vorderes Ende die Mündung der Kanüle dicht verschliesst und die nahe von ihrem vorderen, geschlossenen Ende eine radiale Öffnung aufweist.

Nach einer anderen, besonders bevorzugten Ausführungsart der ersten Variante ist der Durchmesser der vorderen, axialen Mündung der Kanüle kleiner als der Innendurchmesser der Kanüle und das Verschlussmittel ist ein Stopfen, dessen grösster Durchmesser grösser ist als der Durchmesser der vorderen, axialen Mündung der Kanüle. Wenn die Mündung durch den Stopfen verschlossen ist, steht der gesamte Innenquerschnitt der Kanüle für den Durchfluss des Knochenzements zur Verfügung, so dass bei dieser Ausführungsart der Durchflusswiderstand für den Knochenzement wesentlich geringer ist als bei der vorher erwähnten Ausführungsart.

Gemäss einer zweiten Variante der Erfindung wird diese Aufgabe dadurch gelöst, dass eine an ihrem vorderen Ende geschlossene Innenkanüle, vorgesehen ist, die nahe dem vorderen, geschlossenen Ende eine radiale Öffnung für den Austritt des Knochenzements aufweist und die zumindest in einem Bereich nahe dem von ihrem vorderen Ende entfernten Rand der radialen Öffnung satt in die vordere, axiale Mündung der Kanüle passt, wobei die Innenkanüle nach dem Entfernen des Führungsdrahtes so weit in die Kanüle einführbar ist, dass deren radiale Öffnung die Kanüle überragt.

Besondere Ausführungsarten der Erfindung werden nachstehend unter Bezugnahme auf die beiliegenden Zeichnungen beispielsweise erläutert.
Es zeigen:
Figuren 1a bis 1d ein erstes Ausführungsbeispiel einer erfindungsgemässen Vorrichtung mit einer Kanüle mit radialer Öffnung und einer Innenkanüle, die ebenfalls eine radiale Öffnung aufweist,
Figuren 2a bis 2f ein zweites Ausführungsbeispiel einer erfindungsgemässen Vorrichtung, bei dem die Kanüle eine radiale Öffnung aufweist und vome durch einen Körper verschlossen wird, und
Figuren 3a bis 3d ein drittes Ausführungsbeispiel einer erfindungsgemässen Vorrichtung, bei dem die Kanüle vome durch eine Innenkanüle mit radialer Öffnung überragt wird.

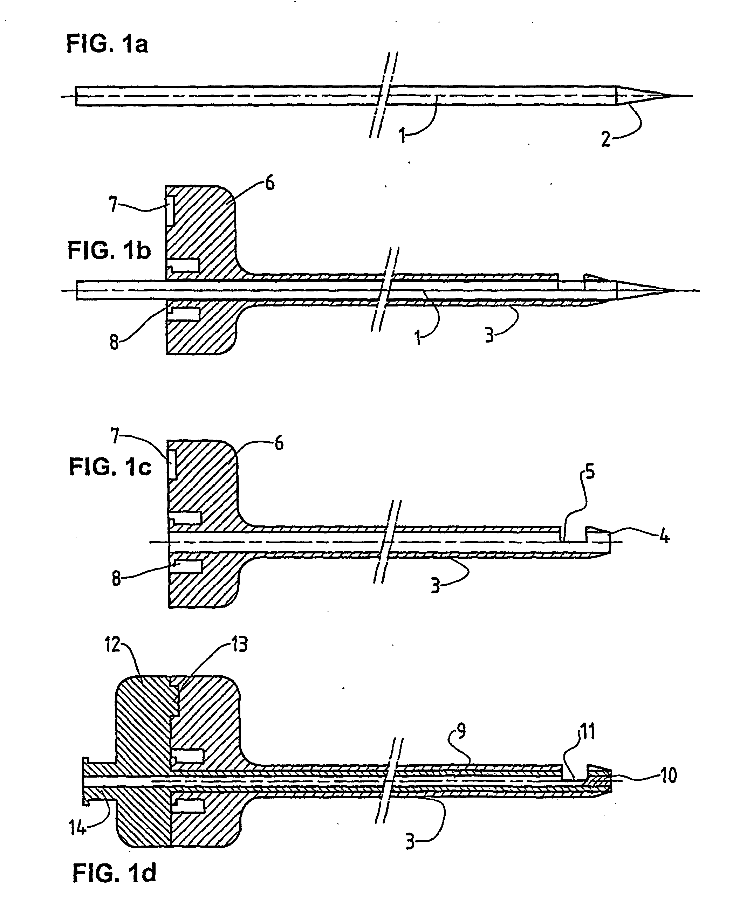

Die Figuren 1a, 2a und 3a zeigen jeweils einen Führungsdraht 1, der an seinem vorderen Ende 2 zugespitzt ist. Dieses vordere Ende 2 des Führungsdrahtes wird unter Röntgenkontrolle im Knochen bis einige Millimeter über die Stelle hinaus vorgetrieben, an welcher der Knochenzement injiziert werden soll. Der Führungsdraht hat typischerweise einen Durchmesser von 2.5 mm und eine Länge von 180 mm, jedoch soll die Erfindung nicht auf diese Abmessungen beschränkt sein.

Nach dem Ausführungsbeispiel gemäss den Figuren 1a bis 1d wird eine Kanüle 3 über den Führungsdraht geschoben. Diese Kanüle 3 weist an ihrem vorderen Ende eine Mündung 4 mit einem scharf geschliffenen Umfangsrand auf, deren Innendurchmesser satt über den Führungsdraht passt. Durch diese Gestaltung wird verhindert, dass beim Vorschieben der Kanüle 3 Knochengewebe ins Innere der Kanüle 3 geraten kann. Am hinteren, in der Zeichnung linken Ende der Kanüle ist ein Griff 6 angeordnet. Die Mündung 4 der Kanüle kann leicht eingezogen sein und der übrige Innendurchmesser der Kanüle kann beispielsweise 3.1 mm betragen. Der Aussendurchmesser der Kanüle 3 kann beispielsweise 4 mm betragen und deren Länge bis zum Griff 130 mm. Nahe bei der Mündung 4 ist in der Kanüle 3 eine radiale Öffnung 5 angeordnet, deren Breite etwas kleiner ist als der Innendurchmesser der Kanüle. Die Länge dieser radialen Öffnung 5 ist mindestens gleich wie jene der Öffnung weiter unten beschriebenen Innenkanüle 9. Der Griff 6 ist asymmetrisch ausgebildet, beispielsweise mit einer zeigerartigen Form, deren Spitze mit der radialen Öffnung 5 ausgerichtet ist, so dass der Chirurg jederzeit die Winkellage der radialen Öffnung 5 kennt. Zudem weist der Griff 6 eine Ausnehmung 7 und eine Kupplung 8 auf, deren Funktion weiter unten erläutert wird. Figur 1c zeigt die Kanüle nach dem Herausziehen des Führungsdrahtes 1.

In Figur 1d ist eine Innenkanüle 9 in die Kanüle 3 eingeführt. Der Aussendurchmesser dieser Innenkanüle ist beispielsweise 3.0 mm, so dass sie mit Spiel in die Kanüle 3 geschoben werden kann. Der Innendurchmesser der Innenkanüle kann beispielsweise 2.5 mm betragen. Das vordere Ende 10 der Innenkanüle ist geschlossen und die Länge der Innenkanüle 9 sowie der Aussendurchmesser des Endes 10 sind so bemessen, dass bei vollständig eingeführter Innenkanüle 9 die Mündung 4 der Kanüle 3 dicht verschlossen ist. Am Hinteren Ende der Innenkanüle 9 befindet sich ein Griff 12, der ebenso wie der Griff 6 der Kanüle 3 asymmetrisch gestaltet ist. Der Griff 12 weist eine Erhebung 13 auf, die zusammen mit der am Griff 6 vorgesehenen Ausnehmung 7 eine Rastvorrichtung bildet. Femer weist die Innenkanüle 9 in der Nähe des Endes 10 eine radiale Öffnung 11 auf, die sich bei eingerasteter Rastvorrichtung 7, 13 mit der Öffnung 5 der Kanüle deckt. Die Breite der radialen Öffnung 11 der Innenkanüle ist etwas geringer als deren Innendurchmesser und die Länge der Öffnung 11 ist so bemessen, dass der Austrittsquerschnitt der Öffnung mindestens gleich gross ist wie der Innenquerschnitt der Innenkanüle 9. Eine am Griff 12 vorgesehene Kupplung 14 dient zum Ansetzen einer Knochenzementquelle, beispielsweise einer Spritze.

Eine Knochenzement-Injektion mit dieser ersten Ausführungsart der erfindungsgemässen Vorrichtung läuft wie folgt ab. Zuerst wird der Führungsdraht 1, wie oben erwähnt, eingetrieben. Dann wird die Kanüle 3 über den Führungsdraht 1 geschoben und so weit eingepresst, bis ihre radiale Öffnung 5 an der Stelle sitzt, an der die Injektion erfolgen soll. Nun wird der Führungsdraht 1 herausgezogen und die Innenkanüle 9 wird in die Kanüle 3 eingeführt. Nachdem die beiden Griffe 6 und 12 mittels der Rastvorrichtung 7, 13 miteinander verriegelt sind und eine Spritze mit Knochenzement mit der Kupplung 14 verbunden ist, kann die Injektion beginnen. Dank der erfindungsgemässen Gestaltung der Vorrichtung ist der Arzt in der Lage, die Austrittsrichtung des Knochenzements auch während der Injektion in einem Bereich zu steuern, indem er die beiden miteinander verriegelten Griffe 6 und 12 und damit auch die beiden miteinander fluchtenden radialen Öffnungen 5 und 11 dreht.

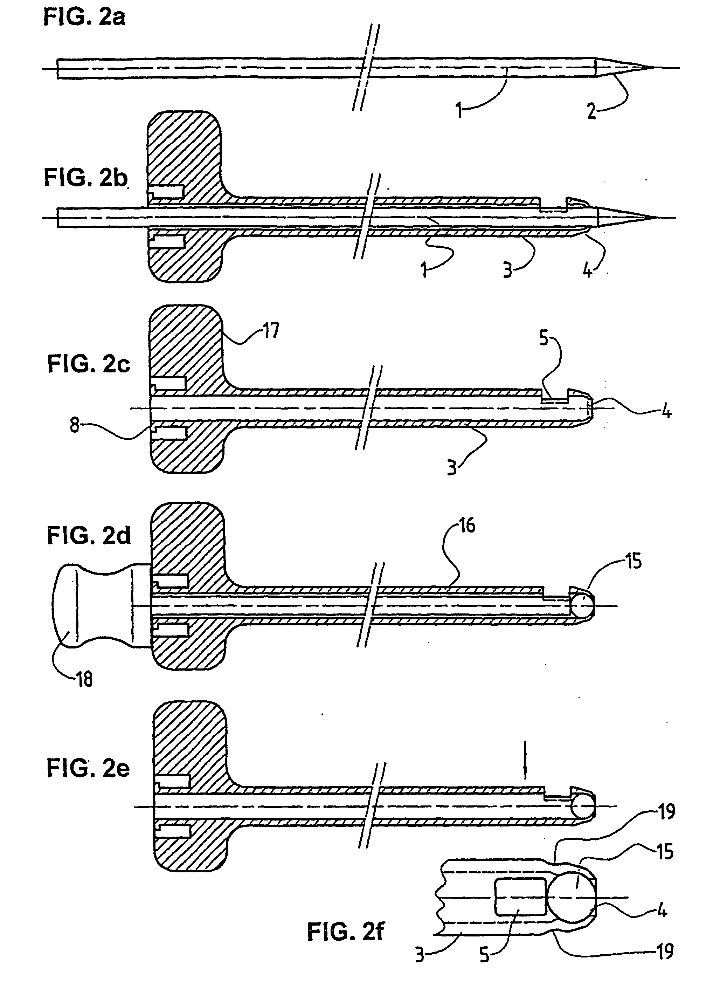

Die Figuren 2a bis 2f zeigen eine zweite Ausführungsart der erfindungsgemässen Vorrichtung, wobei in Figur 2a wiederum ein Führungsdraht 1 mit einer Spitze 2 dargestellt ist. Die Kanüle 3 weist prinzipiell den selben Aufbau auf wie jene beim oben beschriebenen ersten Ausführungsbeispiel, weshalb hier die selben Bezugszeichen verwendet wurden. Ein Unterschied besteht darin, dass bei der Kanüle gemäss den Figuren 2b bis 2e der Griff 17 anders geformt ist als der Griff 7 nach den Figuren 1 b bis 1 d und insbesondere keine Vertiefung 7 aufweist, weil bei diesem zweiten Ausführungsbeispiel keine Innenkanüle 9 vorhanden ist. Die Mündung 4 der Kanüle 3 wird bei dieser Ausführungsart mit einer Kugel 15 verschlossen, welche nach dem Zurückziehen des Führungsdrahtes 1 mit einem Stössel 16 an ihren Platz geschoben wird, wie dies in Figur 2d dargestellt ist. Der Stössel 16 hat einen Halter 18 und ist so lang, dass die Kugel 15 am richtigen Platz sitzt, wenn der Halter 18 am Griff 17 der Kanüle 3 ansteht. Bei diesem Ausführungsbeispiel ist es zwingend, dass die Mündung 4 der Kanüle leicht eingezogen ist. der Durchmesser der Kugel 15 ist leicht kleiner als der Innendurchmesser der Kanüle3, aber etwas grösser als der Innendurchmesser der Mündung 4 und auch grösser als die Breite der radialen Öffnung 5. Somit ist gewährleistet, dass die Kugel 15 leicht in die Kanüle 3 eingeführt werden kann, aber nicht durch die radiale Öffnung 5 oder die Mündung 4 austreten kann. Damit die Kugel 15 nicht ungewollt zurückrollen kann, nachdem sie durch den Stössel 16 an ihren Platz geschoben wurde, sind in der Wandlung der Kanüle 3 kleine Einbuchtungen 19 vorgesehen, wie dies in Figur 2f zu sehen ist. Die Einbuchtungen sind so bemessen, dass Beim Einschieben der Kugel 15 ein gewisser Widerstand überwunden werden muss.

Diese zweite Ausführungsart der erfindungsgemässen Vorrichtung hat gegenüber den anderen Ausführungsarten den grossen Vorteil, dass keine Innenkanüle erforderlich ist und demzufolge der gesamte Innenquerschnitt der Kanüle 3 für das Fliessen des Knochenzements zur Verfügung steht. Dies hat beim Injizieren einen wesentlich geringeren Druckverlust zur Folge.

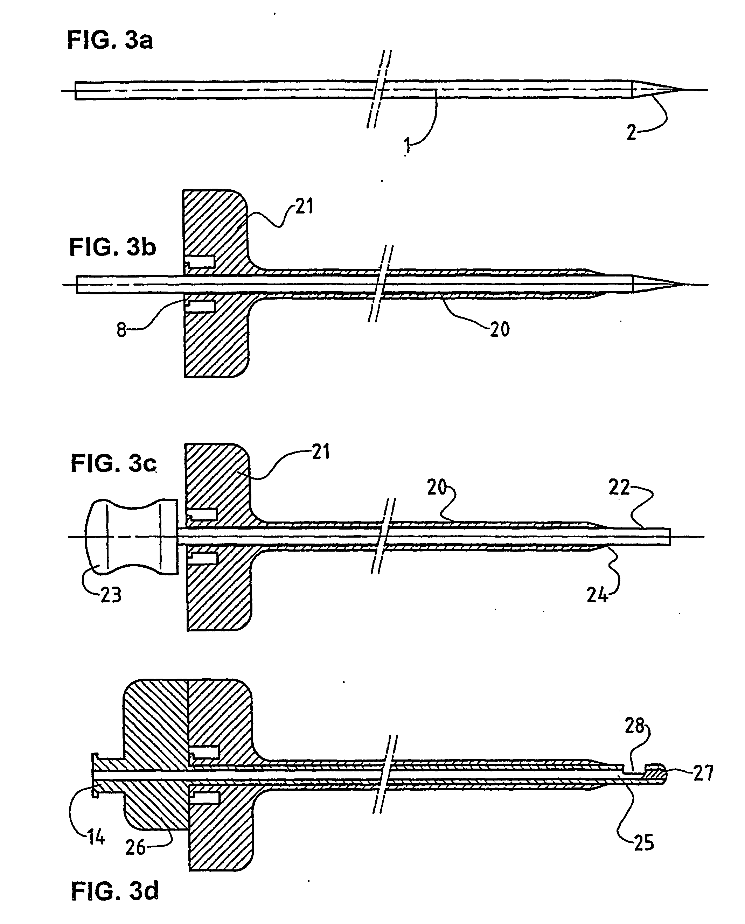

Beim in den Figuren 3a bis 3d dargestellten dritten Ausführungsbeispiel ist ebenfalls ein Führungsdraht 1 vorgesehen, wie dies aus der Figur 3a ersichtlich ist. Die Kanüle hat hier die Bezugszahl 20 und unterscheidet sich von den vorher beschriebenen Kanülen dadurch, dass sie keine radiale Öffnung hat. Deshalb ist auch der Griff 21 der Kanüle 20 nicht asymmetrisch. Figur 3c zeigt einen Obturator 22, mit dem der Arzt nach dem Einführen der Kanüle 20 ausserhalb deren Mündung 24 Platz schafft, um anschliessend die Innenkanüle 25 einführen zu können, wie dies in Figur 3d dargestellt ist. Der Obturator 22 hat einen Kopf 23 und seine Länge ist so bemessen, dass wenn der Kopf 23 am Griff 21 der Kanüle ansteht, sein vorderes Ende so weit aus der Mündung 24 ragt wie später die Innenkanüle 25. Die Innenkanüle 25 hat nahe bei ihrem geschlossenen Ende 27 eine radiale Öffnung 28 für den Austritt des Knochenzements. Am anderen Ende weist die Innenkanüle 25 einen Griff 26 auf, der derart asymmetrisch ausgebildet ist, dass an seiner Stellung die Lage der radialen Öffnung 28 gesehen werden kann.

Mit dieser dritten Ausführungsart der Erfindung verläuft eine Knochenzement-Injektion anfänglich gleich wie mit den beiden anderen Ausführungsarten, indem zuerst der Führungsdraht 1 eingetrieben und dann die Kanüle 20 über den Führungsdraht 1 geschoben wird. Nach dem Entfernen des Führungsdrahtes 1 wird mit dem Obturator 22 im Bereich vor der Mündung 24 das Knochengewebe so weit zusammengeschoben und komprimiert, dass anschliessend die Innenkanüle 25 ohne zu grossen Widerstand eingeführt werden kann. Nach dem Einführen der Innenkanüle 20 wird deren radiale Öffnung durch Drehen des Griffs 26 wunschgemäss ausgerichtet und mit einer mit der Kupplung 14 verbundenen Spritze der Knochenzement injiziert.

Alle drei beschriebenen Ausführungsarten der erfindungsgemässen Vorrichtung erlauben somit ein exaktes Plazieren der Knochenzementfüllung, indem die Austrittsrichtung des Knochenzements durch drehen der betreffenden Kanüle bestimmt und sogar während der Injektion verändert werden kann.

Damit sowohl die Kanüle 3 nach dem ersten Ausführungsbeispiel als auch die Kanüle 20 nach dem dritten Ausführungsbeispiel auch zum axialen injizieren von Knochenzement verwendet werden können, weisen deren Griffe 6 und 21 eine Kupplung 8 zum direkten Ansetzen einer Spritze auf. Dadurch wird das Sortiment an Kanülen klein gehalten.

## Patentansprüche

1. Vorrichtung zum Injizieren von Knochenzement, enthaltend einen Führungsdraht (1) und eine zumindest mit dem Innendurchmesser ihrer vorderen, axialen Mündung (4) satt auf den Führungsdraht (1) passende Kanüle (3), **dadurch gekennzeichnet, dass** Verschlussmittel (9, 15) vorhanden sind, mit denen die vordere, axiale Mündung (4) der Kanüle (3) nach dem Entfemen des Führungsdrahtes (1) verschliessbar ist und dass die Kanüle (3) nahe der vorderen, axialen Mündung (4) eine radiale Öffnung (5) für den Austritt des Knochenzements aufweist.

2. Vorrichtung nach Anspruch 1, **dadurch gekennzeichnet, dass** das Verschlussmittel eine an ihrem vorderen Ende geschlossene Innenkanüle (9) ist, die so in die Kanüle (3) einführbar ist, dass ihr vorderes Ende (10) die Mündung (4) der Kanüle (3) dicht verschliesst und die nahe von ihrem vorderen, geschlossenen Ende (10) eine radiale Öffnung (11) aufweist.

3. Vorrichtung nach Anspruch 1, **dadurch gekennzeichnet, dass** der Durchmesser der vorderen, axialen Mündung der Kanüle kleiner ist als der Innendurchmesser der Kanüle und dass das Verschlussmittel ein Stopfen (15) ist, dessen grösster Durchmesser grösser ist als der Durchmesser der vorderen, axialen Mündung (4) der Kanüle.

4. Vorrichtung nach Anspruch 3, **dadurch gekennzeichnet, dass** der Stopfen (15) die Form einer Kugel hat.

5. Vorrichtung zum injizieren von Knochenzement, enthaltend einen Führungsdraht (1) und eine zumindest mit dem Innendurchmesser ihrer vorderen, axialen Mündung (24) satt auf den Führungsdraht passende Kanüle (20), **gekennzeichnet durch** eine an ihrem vorderen Ende (27) geschlossene Innenkanüle (25), die nahe dem vorderen, geschlossenen Ende (27) eine radiale Öffnung (28) für den Austritt des Knochenzements aufweist und die zumindest in einem Bereich nahe dem von ihrem vorderen Ende entfernten Rand der radialen Öffnung (28) satt in die vordere, axiale Mündung (24) der Kanüle passt, wobei die Innenkanüle (25) nach dem Entfemen des Führungsdrahtes (1) so weit in die Kanüle (20) einführbar ist, dass deren radiale Öffnung die axiale Mündung (24) der Kanüle überragt.

## Claims

1. Device for injecting bone cement, containing a guide wire (1) and a cannula (3) fitting snugly on the guide wire (1) at least with the inner diameter of its front axial orifice (4), **characterised in that** there are dosing off means (9, 15) with which the front axial orifice (4) of the cannula (3) is closable after removal of the guide wire (1), and **in that** the cannula (3) has a radial aperture (5), near the front, axial orifice (4), for exit of the bone cement.

2. Device according to claim 1, **characterised in that** the closing off means is an inner cannula (9) closed at its front end, which inner cannula is insertable into the cannula (3) in such a way that its front end (10) tightly closes off the orifice (4) of the cannula (3) and has a radial aperture (11) near its front, closed end (10).

3. Device according to claim 1, **characterised in that** the diameter of the front, axial orifice of the cannula is smaller than the inner diameter of the cannula and **in that** the closing off means is a stopper (15) the largest diameter of which is greater than the diameter of the front, axial orifice (4) of the cannula.

4. Device according to claim 3, **characterised in that** the stopper (15) has the shape of a ball.

5. Device for injecting bone cement, containing a guide wire (1) and a cannula (20) fitting snugly on the guide wire at least with the inner diameter of its front axial orifice (24), **characterised by** an inner cannula (25), closed at its front end (27), which inner cannula has near the front closed end (27) a radial aperture (28) for the exit of the bone cement, and which, at least in a region near the edge remote from its front end, of the radial aperture (28) fits snugly in the front axial orifice (24) of the cannula, whereby, after the removal of the guide wire (1), the inner cannula (25) is insertable so far into the cannula (20) that its radial aperture extends beyond the axial orifice (24) of the cannula.

## Revendications

1. Dispositif pour injecter du ciment osseux, qui contient un fil de guidage (1) et une canule (3) ajustée au moins par le diamètre interne de son embouchure axiale avant (4) affleure sur le fil de guidage (1), **caractérisé en ce que** des moyens de fermeture (9, 15) sont prévus à l'aide desquels l'embouchure avant axiale (4) de la canule (3) peut être fermée après le retrait du fil de guidage (1), **en ce que** la canule (3) présente près de l'embouchure axiale avant (4) une ouverture radiale (5) pour la sortie du ciment osseux.

2. Dispositif selon la revendication, **caractérisé en ce que** le moyen de fermeture est une canule interne (9) fermée sur son extrémité avant qui peut être insérée dans la canule (3) de sorte que son extrémité avant (10) ferme de manière étanche l'embouchure (4) de la canule (3) et qui présente proche de son extrémité fermée avant (10) une ouverture radiale (11).

3. Dispositif selon la revendication 1, **caractérisé en ce que** le diamètre de l'embouchure axiale avant de la canule est inférieure au diamètre interne de la canule et **en ce que** le moyen de fermeture est un bouchon (15) dont le plus grand diamètre est plus grand que le diamètre de l'embouchure axiale avant (4) de la canule.

4. Dispositif selon la revendication 3, **caractérisé en ce que** le bouchon (15) présente la forme d'une sphère.

5. Dispositif pour l'injection de ciments osseux qui contient un fil de guidage (1) et une canule (20) ajustée au moins par le diamètre interne de son embouchure axiale avant (24) affleure sur le fil de guidage, **caractérisé par** une canule interne (25) fermée à son extrémité avant (27), qui présente proche de son extrémité avant fermée (27) une ouverture radiale (28) pour l'extraction du ciment osseux et qui est ajusté au moins dans une zone proche du bord éloigné de son extrémité avant de l'ouverture radiale (28), dans l'embouchure axiale avant (24) de la canule, la canule interne (25) étant insérable après le retrait du fil de guidage (1) suffisamment loin dans la canule (20) pour que son ouverture radiale dépasse l'embouchure axiale (24) de la canule.
